# EUROPEAN PATENT APPLICATION

(11) **EP 1 287 851 A1**
(43) Date of publication of application: **05.03.2003**
(21) Application number: 02255954.6
(22) Date of filing: 28.08.2002
(51) Int. Cl.: A61N 1/375

(54) **Method of producing textured surfaces on medical implants**

(30) Priority: 28.08.2001 US 315271 P
(71) Applicant: Robinson, Mark L., Berks County, PA (US); Staab, Michael L., Lancaster County, PA (US)
(72) Inventor: Robinson, Mark L., Berks County, PA (US); Staab, Michael L., Lancaster County, PA (US)
(74) Representative: Wilson Gunn M'Caw

(57) **Abstract**

Methods of texturizing medical implants are provided which involve embossing the surface of these implants to create a textured pattern. Preferred roll embossing techniques are disclosed for improving scratch resistant properties, minimizing glare, improving lubricant retention and/or creating random or uniform patterns on medical implants, such as the outer shield of pacemakers and defibrillators, as well as orthopedic implants.

## Description

### FIELD OF THE INVENTION

This invention relates to a method of embossing strip material used for medical implants, and more particularly, to methods of producing textured surfaces on implant quality titanium strip.

### BACKGROUND OF THE INVENTION

Medical implant applications, such as implantable pacemakers, defibrillators, drug infusion pumping devices, and orthopedic implants, are commonly made from, or housed within, corrosion-resistant metal, such as titanium, titanium alloys, nickel alloys or stainless steel.

Medical implants have often been finished by a variety of techniques, including hand polishing, media blasting, and electrolytic and chemical polishing. It is difficult to effectively polish all surfaces of medical implants, since they often have small features and intricately curved surfaces. Additionally, mechanical and electrolytic polishing can produce a surface finish that is bright and light reflective. Such a surface can sometimes lead to glare under the bright light of a surgical procedure, and reveal scratches and blemishes which are, at a minimum, aesthetically undesirable, and which can sometimes lead to a part's rejection on purely cosmetic grounds.

Matte or "scratch resistant" surfaces have been produced by chemical polishing and media blasting techniques. Such surfaces are in demand, since they are less light-reflective and conceal scratches and blemishes, rather than literally resisting them as the term "scratch resistant" suggests.

Recent artisans have attempted to create matte surfaces on medical implants. Baswell et al., U.S. Patent No. 4,704,126, issued November 3, 1987, discloses a method of chemically polishing medical implants by immersing them in a mixed acid solution to produce a smooth, matte surface. This acid polishing technique is principally designed to minimize tissue fixation on titanium or titanium alloy medical implants, while preventing reflective glare from interfering with surgical procedures. While smoothing the exterior of medical implants offers some advantages, such surfaces do not conceal scratches and blemishes well enough, and are not known to retain metal working lubricants in any significant way during metal working processes, which is a disadvantage in deep drawing metal working operations. In addition, acid baths generate a considerable amount of hazardous waste, and require time consuming cleaning, washing and acid neutralization steps in the manufacture of medical implants.

Johnson, U.S. Patent No. 5,673,473, issued October 7, 1997, described a method of creating a scratch resistant surface on a medical implant shield, by blasting the titanium metal strip precursor with metallic media. The '473 patent reports that metallic media blasting enhances scratch resistant properties, while simultaneously improving manufacturing "throughput" without sacrificing shield biocompatability. Despite the teachings of this patent, metallic media blasting always presents a chance that embedded media will unintentionally be retained on the implant surface. Media blasting generally forms the same texture on all surfaces of the metal strip and is a relatively slow operation to perform. Media blasting is also incapable of controlling the nature of the texture, such as the degree of roughness, randomness or orientation.

Accordingly, there remains a need for providing textured surfaces on implant quality metal strips which improves manufacturing "throughput", produces a high quality scratch resistant surface, and improves control over the nature of the texture imparted onto the medical implant surface.

### SUMMARY OF THE INVENTION

In a first embodiment, the present invention provides a method of manufacturing an outer shield of a medical implant, which includes providing a sheet metal substrate having first and second planar surfaces thereon. The method further includes embossing the sheet metal substrate to provide an embossed sheet metal substrate having a textured pattern on at least the first surface, and forming the embossed sheet metal substrate into an outer shield exhibiting said textured pattern on at least an external facing surface portion for helping to conceal small surface defects thereon.

The present invention provides surface defect concealment on medical implants, and especially outer shields of pacemakers and defibrillators, and other implantable devices. The embossing processes of this invention are more expedient than media blasting or chemical polishing. For example, the embossing step of this invention can provide a textured surface on a medical grade titanium strip at speeds often to fifty times faster than media blasting. Since no particulate media is used in the preferred embossing steps to form the surface texture, there is virtually no chance of embedding media or foreign objects in the surface of the implant. While media blasting forms an identical texture on either side of the strip, the embossing processes of this invention allow for differing textures on different locations on the strip by utilizing, for example, upper and lower rolls with different engraving patterns. Additionally, while media blasting is, by its very nature, a random process generating a random texture, the embossing processes of this invention can generate either a random, regular, periodic, or semi-periodic pattern on the strip, as desired. Patterns simulating wood, tweed, leather, and stuccos, can be engraved on titanium, titanium alloy, nickel alloy and stainless steel metal strips, as desired. More preferably, a non-directional, non-reflective surface texture which mimics media blasting, is used. Finally, no hazardous waste is generated by embossing, since no acid baths are required. The textured surfaces produced on the metal surfaces of this invention conform merely to the engraved tool design and the degree of pressure selected.

In further embodiments of this invention, medical implants are provided having embossed textured patterns which retain lubricants to minimize galling in subsequent metal forming operations. Medical implants with textured embossed surfaces are also provided which have different engraving patterns on their surfaces. For example, a simulated leather grain can be produced on the exterior of the shield, with a stucco-like pattern on the interior of the shield, so as to readily distinguish these surfaces during subsequent manufacturing operation. Additionally, the embossed patterns of this invention can be designed to enhance tissue implantation on the surface of the medical implant, when desirable, such as in bone ingrowth applications of orthopedic implants. Patterns can be developed which retain more or less metal working lubricant, such as oil or detergent-based lubricants, for deep drawing, stamping or shaping operations.

### BRIEF DESCBIPTIONS OF THE DRAWINGS

The accompanying drawings illustrate preferred embodiments of the invention, as well as other information pertinent to the disclosure, in which:
FIG. 1: is a flow diagram of a preferred manufacturing method for producing an outer shield of a medical implant;
FIG. 2: is a flow diagram of an alternative manufacturing method for producing an outer shield of a medical implant;
FIG. 3: is a front perspective view of a roll embossing machine in the process of engraving a metal strip;
FIG. 4: is a top planar view of a bottom hardened steel engraving roll embodiment of this invention.

### DETAILED DESCRIPTION OF THE INVENTION

This invention provides improved metal finishing techniques for producing textured surfaces on medical implants, such as for example, the outer shield of pacemakers and defibrillators. This invention is also applicable to shields or cases for implantable medication dispensing devices, or the surface of orthopedic implants, stents, plates, orthopedic screws and any number of medical devices used in contact with the human body. As used herein, the following terms are defined:

"Sheet or strip metal" means a substantially planar thin gauge metallic substrate having a thickness of less than about 3/16"(4.76mm) inches;

"Roll embossing" means a three-dimensional texturizing process involving one or more engraved rolls.

"Embossing" means any method useful in providing a pattern or shape to a metal sheet material, including one-sided embossing (coining), roll forming of deep ridges (corrugation) and rotary or roll embossing.

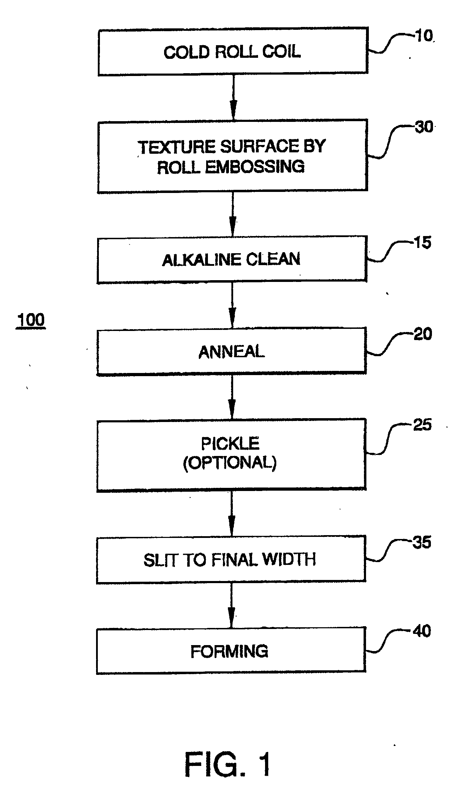

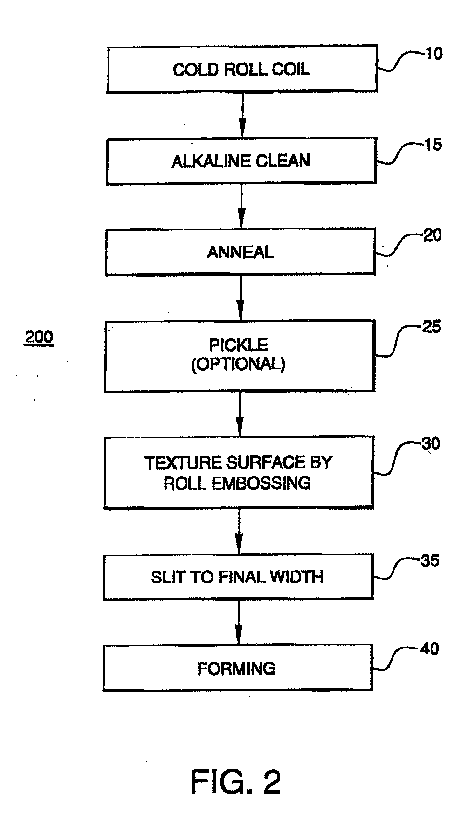

With reference to the figures, and in particular FIGS. 1 and 2 thereof, there is shown a pair of manufacturing sequences 100 and 200 for texturing the implant quality metal strips of this invention. While these preferred steps can be used in a different order, the disclosed sequence embodiments 100 and 200, provide a favorable combination of texture and metal properties.

Sequence 100 begins with a source of strip metal, such as a cold rolled coil of titanium, titanium alloy, nickle, nickel alloy or stainless steel. Titanium and its alloys are often used in corrosive environments, as in contact with body fluid, such as blood. Titanium has a light weight, high strength-to-weight ratio, and non-magnetic properties. Depending on the predominate phase or phases in the microstructure, titanium alloys are categorized as alpha, alpha-beta, and beta. This natural grouping not only reflects basic titanium production metallurgy, but also indicates general properties peculiar to each type. Chemically pure ("CP") titanium and Ti-6Al-4V alloys are commonly selected for medical implant applications, since they are extremely biocompatable materials. While CP titanium may contain small amounts (<1 wt%) of O₂ or iron, it is an alpha alloy type having a coefficient of thermal expansion of 5.4x10⁻⁶ in/in-°F, within the range of 32-1000°F, and a tensile modulus of elasticity of about 14.9x10⁶psi. Ti-6Al-4V is an alpha-beta alloy type, having a coefficient of thermal expansion of about 3.9x10⁶ in/in-°F over the same temperature range, and a tensile modulus of elasticity of about 16.5x10⁶psi. CP titanium has excellent corrosion resistance and excellent ductility for maximum formability during the drawing of medical implant shields. Ti-6Al-4V is one of the more versatile titanium alloys, and is used in many corrosion resistant applications. It has much greater electrical resistivity, having a RT electrical resistivity of 171 micro-ohms-cm, whereby the high purity titanium has a RT electrical resistivity of 56 micro-ohms-cm. Other titanium alloys that are useful for this invention include Ti-15Mo-2.7Nb-3Al-0.2Si, beta-21S alloy, which is an ideal candidate for orthopedic implants, due to its extremely low hydrogen uptake efficiency levels. Like stainless steel, which is also a candidate for this invention, titanium sheet work hardens significantly during forming, even during media blasting and embossing. Minimum bend-radius rules are nearly the same for both, although spring back is greater for titanium. CP grades of heavy titanium plate are cold formed or, for more severe shapes, warm formed at temperatures of about 800°F. Alloy grades can be formed at temperatures as high as 1400°F in inert gas atmospheres.

Despite their high strength, some alloys of titanium have superplastic characteristics in the range of 1500-1700°F. The alloy used for most superplastically formed parts is the standard Ti-6Al-4V alloy.

Selected titanium alloys useful in the methods of this invention are disclosed below in Table I.

**Table I:**

| **Titanium alloys properties** | | | | | |
|---|---|---|---|---|---|
| **Nominal alloy composition** | **Yield Strength (10**^{**3**}**) Minimum at room temp.** | **Tensile Modulus of Elasticity (10**^{**6**} **psi)** | **Coef. of thermal expansion (32-1,000°F) (10**^{**-6**}**in./in.-°F)** | **RT Thermal conductivity (Btu-ft/h-ft**^{**2**}**-°F)** | **RT Electrical resistivity (µohm-cm)** |
| *Ti(high purity)(Alpha) | 25 | 14.9 | 5.4 | 9.0 | 56 |
| *Ti (plus O₂,F)(Alpha) | 70 | 15.1 | 5.4 | 9.8 | 60 |
| Ti-0.2Pd (Alpha) | 40 | 14.9 | 5.4 | 9.5 | 56 |
| Ti-5Al-2.5Sn (Alpha) | 115 | 16.0 | 5.3 | 4.5 | 157 |
| Ti-6Al-2Sn-4Zr-2Mo (Alpha) | 120 | 18.5 | 5.6 | ― | 199 |
| Ti-6Al-4V (Alpha-beta) | 120 | 16.5 | 3.9 | 3.9 | 171 |
| †Ti-6Al-4VEL1 (Alpha-beta) | 120 | 16.5 | 5.6 | 4.2 | 171 |
| Ti-3Al-SV-6Cr-4Zr-4Mo (Beta) | 160 | 15.0 | 5.4 | ― | ― |
| Ti-15Mo-3Nb-3Al-0.2Si (Beta) | 160 | 15.5 | 4.9 | 4.4 | 135 |

| | | | | | |
|---|---|---|---|---|---|
| *Also available with 0.05% max Fe for superior corrosion resistance. | | | | | |
| †Develops excellent fracture toughness properties with special mill processing. | | | | | |

In the preferred manufacturing sequences 100 and 200, cold rolled coil of titanium, titanium alloy, nickel, nickel-alloy or stainless steel is provided at cold rolled coil step 10. The strip within the coil should be pre-rolled to a thickness of about .005-.040 in, preferably about .010-.020 in, with a target thickness of about .012 in. The strip material within the coil is used generally, especially in the manufacture of implantable medical device shields, due to its high strength, ductility, fracture resistance, biocompatability and corrosion resistance. However, if the manufacturing methods of this invention are used with thicker substrates, such as for medical implants, the roll embossing techniques of this invention can be provided with larger nip spacing to enable larger materials to be texturized.

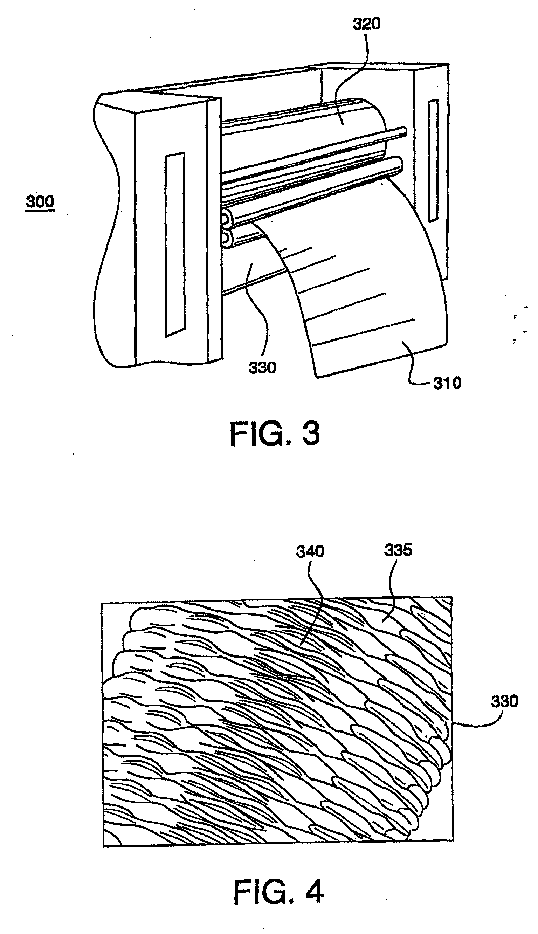

In the manufacturing sequence 100 of FIG. 1, the cold roll strip is embossed at roll embossing step 30. While it is anticipated that other embossing technique could be useful for certain end-uses associated with this invention, such as single-sided embossing (coining), for thicker substrates, roll forming for deep ridges, for added strength, roll embossing is the preferred technique for medical device shields. Ideally, the embossing machine 300 shown in FIG. 3 is situated directly after an uncoiler in the processing line and may be followed by a number of different operations. Typically, these operations include recoiling, slitting or cutting-to-width, slitting, roll forming, stamping, or any combination thereof.

The preferred embossing machine 300 can either be a stationary fixture in the metal processing line, or it can be made movable with wheels, rails, or a crane and lifting bolt assembly. While most machines are driven with an integral motor and drive package, embossing can also be performed using the power of a recoiler or other device and an unpowered pull-through embossing stand. Horsepower requirements depend on line speed and, to a lesser degree, material thickness, pattern, and roll size.

Located within the embossing stand of the preferred embodiment are two engraved and mated hardened steel rolls 320 and 330, geared together to maintain top-to-bottom pattern registration. The engraved metal embossing rolls 320 and 330 are preferably manufactured from high quality 52100 modified steel forgings, through hardened to 62-65 Rockwell C. While this may take a little more time and be slightly higher in cost, it yields dividends in increased longevity and wear. The width and diameter of these rolls 320 and 330 depends on the strip width, material thickness, pattern depth, and material tensile strength and hardness.

In FIG. 4 a preferred embossing roll 330 is shown with protuberances 340 and flat regions 335. While not presently committed to any particular pattern, leaving flat areas in the engraved roll can preserve the original sheet thickness in certain areas to maintain full mechanical strength. One or both rolls 320 and 330 can be engraved with a common or different pattern.

The engraved roll journals are housed in a bearing and block assembly (not shown). In most machines, the upper roll blocks are stationary, while the bottom roll blocks are movable. The pressure with which the bottom roll is raised is referred to as the tonnage capacity. This figure also depends on the aforementioned parameters.

Embossing machines are generally sized to give 2" of strip clearance on each side of an engraved embossing roll. However, each unit is custom-manufactured, so there are no standard widths. In fact, machines less than 6 wide and more than 76" wide are currently in operation.

The reasons for employing embossed metal for medical devices can be divided into two distinct categories: aesthetic and functional. Many applications serve both purposes.

Aesthetic uses of embossing are those which enhance the appearance of a product, such as the elimination of glare. By creating a series of small peaks and valleys on the implant's shield, small surface defects, such as scratches and blemishes can also be more effectively concealed.

The embossed medical implant applications of this invention that begin as aesthetic, such as scratch concealment, could very well end up with functional improvements. Functional applications of embossing include those in which a performance characteristic is enhanced, and can involve, for example, better liquid dispersion and greater friction and static reduction, as in better metalworking lubricant retention during drawing and other metal forming operations. Deep textures on the outside of the shield can also increase thermal conductivity by increasing the surface area. Additionally, texturing the exterior can encourage tissue attachment. Embossed patterns can also improve stiffness and rigidity which improves the shield's toughness, stiffness and impact strength.

One additional benefit of increased stiffness is the ability to reduce weight and material to save on material costs. Reduction of "oil canning", diffusion of light, and decreased manufacturing rejects are other important side benefits.

The most popular embossed metal patterns include leather grains, wood grains, and stuccos, although almost any pattern can be engraved on the mated set of hardened rolls 320 and 330. In the preferred implants of this invention, a non-directional, non-reflective surface texture is used. Warnings, brand names, installation directions and other instructions can also become a part of the pattern. Creating a new pattern and engraving the rolls are complex and highly skilled tasks. Engravers can take any idea, prototype, or artwork and develop original tooling by methods including electroforming, etching, punching, routing, laser, or computer generated graphic enhancement.

Once tooling has been manufactured, a mill is produced specific to the work roll that will be engraved. This mill displaces an acid-resist coating on the rolls and exposes metal, which is subsequently etched with acid and removed. The entire process is repeated again and again until the full depth (usually about .001-.1 inch) and finish are obtained.

Thus, the pattern on the small mill is transferred to a large hardened work roll. This hardened top roll is then mated with and geared with a bottom roll in the embossing machine. Rolls can be re-engraved on-site as long as the pattern fidelity of the rolls and gear clearance and roll diameter parameters remain adequate.

Three preferred methods for obtaining embossed metal strip for the outer shields of this invention include:
1. Purchase pre-embossed material from a service center, coil coater, or custom embosser;
2. Obtain a set of embossing rolls to be run in a third-party's embossing machine for toll embossing applications; and
3. Procure an embossing machine and roll set for in-house production.

In any of these cases, one can compare the additional cost of outside embossing with the equipment and labor costs associated with inside embossing.

While embossed metal is handled and formed in exactly the same way as its flat counterpart, several conditions are important to keep in mind.

Bending radii and die clearances must take into account the material's actual cross-section thickness versus material thickness only. Some deep draws or severe bends may distort, wash out, or even split open sharp patterns and should, therefore, be avoided (or tested on a small scale before production).

Shallower embossed patterns, preferred by this invention, such as the leather grain family, may affect material flatness and, therefore, need corrective leveling. This is especially true as the product increases in width.

A matched set of embossing rolls will deflect during the embossing process. While this deflection can be compensated for by adding crown to the rolls, this crown is pertinent to one particular gauge (usually the most commonly run). Embossing above or below the target thickness may result in sheet shape and/or pattern appearance anomalies. Again, this is more pronounced with shallower patterns.

While materials more than .040 inch thick can be textured by roll embossing techniques, the result is usually a coined/embossed hybrid. This invention recommends staying with the .005-.040 inch strip thickness range for roll embossing, preferably about .010-.020 inches, with a target thickness of .012 inches. Uniform in-feed tension is desirable while material can exit with or without tension.

In the next step 15, the strip 310 is treated to an alkaline cleaning step 15, prior to an annealing step 20. The alkaline cleaning step 15 removes organic contaminates, such as oil, which reside on the surface of the cold roll coil 10 from forming and handling.

The annealing step 20 is designed to stress relieve the sheet material prior to subsequent processing. It is typically carried out in a vacuum or inert gas atmosphere, such as argon. While the annealing temperature and sequence depends upon the type of alloy used, generally for titanium alloys, a temperature of about 1400-1800°F, preferably solution treated at 1750°F, followed by a water quench or air cool, is acceptable. Titanium alloys can also be aged for up to 4-6 hours at 1000°F.

In addition to the annealing step 20, a pickling step 25 is optionally employed to remove the oxide layer formed during the annealing step, and also to clean the substrate surface without dissolving away the surface layer produced during the cold rolling of the coil 10. Following pickling 25 , the strip is slit to final width at slit step 35, and is then sent to a subsequent forming step 40 (usually an intermediate manufacturer) for final manufacture into a medical implant shield, for example.

Sizing and fitting can also be accomplished after forming for producing the final elements of the medical device shield itself. Sizing and trimming affects subsequent medical device manufacturing processes, such as machining and welding operations.

The forming step 40 of conventional medical device shields, such as cardiac pacemakers, for example, operates by mounting one or more textured strips in accordance with conventional methods. Initially, the strip is used as a blank cut from the coil or strip of embossed titanium sheet. A drawing punch (not shown) forces the blank holder through a cylindrical opening in a dye, such that a half shield is formed from the flat blank. After trimming, this half-shield is mated with another half-shield. The finished medical device can be provided by enclosing the internal electronics and battery cell into the shield halves. The circuitry then can be connected to the feedthroughs. Subsequent electron beam (E B), laser or ultrasonic welding of the shield halves together along their edges forms a substantially hermetic closure. A molded plastic connector block assembly containing electrical connecters for attachment to the feedthroughs is typically installed as a final step.

In the alternative manufacturing sequence 200, shown in FIG. 2, the roll embossing step 30 is moved further down the processing line, after the optional pickling step 25. This provides the additional benefit of being able to emboss the sheet metal after it is softened by the annealing step 20, which may require less tonnage, and may provide greater detail with less effort to print. Since a formed shield, for example, will likely be further annealed prior to final assembly, moving the roll embossing step 30 further on down the manufacturing line, will not lead to a wasted annealing step.

From the foregoing, it can be realized that this invention provides improved methods for manufacturing medical implants having textured surfaces, and more particularly, to outer shields of pacemakers and defibrillators, having a scratch resistant surface manufactured in a fraction of the time currently required by media blasting techniques. It is further possible that roll embossing will provide shield metal that has been strengthened by the textured surface formed to its surface. Additionally, the roll embossing, or other embossing, techniques of this invention can produce designed patterns for increased metal working lubricant retention, decreased glare, and increased thermal conductivity for suitable end-use applications. It is conceivable that the texturing designs of this invention can be combined with more conventional media blasting, chemical, hand or electrolytic polishing to provide surfaces having multiple characteristics and properties on the same implant. Also, the embossing techniques of this invention do not contribute to the contamination of the surface of the implant, and can be performed directly on the metal strip, without significantly changing its planar shape. Although various embodiments have been illustrated, this is for the purpose of describing, but not limiting the invention. Various modifications, which will become apparent to one skilled in the art, are within the scope of this invention.

## Claims

1. A method of manufacturing an outer shield of a medical implant **characterized by**:
(a) providing a sheet metal substrate (310) having first and second planar surfaces thereon;
(b) embossing (30) said sheet metal substrate (310) to provide an embossed textured pattern on said first planar surface; and
(c) forming (40) the embossed sheet metal substrate into an outer shield of said medical implant, said outer shield having an inside and outside surface, whereby said embossed textured pattern is located at least on said outside surface for helping to conceal small surface defects thereon.

2. The method of claim 1 wherein said sheet metal substrate comprises a metal selected from the group containing titanium, nickle, and alloys thereof, and stainless steel.

3. The method of either claim 1 or 2 wherein said textured pattern comprises a non-reflective surface texture.

4. The method of any preceding claim further comprising pickling said sheet metal substrate.

5. The method of any preceding claim further comprising annealing said sheet metal substrate.

6. The method of any preceding claim wherein said forming step (c) forms said sheet metal substrate into an outer shield for a cardiac pacemaker or defibrillator.

7. The method of claim 6 wherein said outer shield has a thickness of less than about 3/16 inches (4.76 mm).

8. A medical implant comprising the outer shield of either claim 6 or 7.
